(19) Europäisches Patentamt
European Patent Office
Office européen des brevets

(11) Veröffentlichungsnummer: **0 037 480**
**B1**

# (12) EUROPÄISCHE PATENTSCHRIFT

(45) Veröffentlichungstag der Patentschrift: **28.09.83**

(51) Int. Cl.³: **C 07 D 233/96**

(21) Anmeldenummer: **81101882.9**

(22) Anmeldetag: **13.03.81**

## (54) Verfahren zur Herstellung von 5-Arylidenhydantoinen (A).

(30) Priorität: **09.04.80 DE 3013626**

(43) Veröffentlichungstag der Anmeldung: **14.10.81 Patentblatt 81/41**

(45) Bekanntmachung des Hinweises auf die Patenterteilung: **28.09.83 Patentblatt 83/39**

(84) Benannte Vertragsstaaten: **BE CH FR GB IT LI NL**

(56) Entgegenhaltungen:
**DE-B-1 038 050**
**FR-A-2 189 040**
**CHEMICAL ABSTRACTS, vol. 82, no. 21, May 26, 1975, Seite 630, Zusammenfassung 140 139v Columbus, Ohio, USA**

(73) Patentinhaber: **Degussa Aktiengesellschaft, Weissfrauenstrasse 9, D-6000 Frankfurt am Main 1 (DE)**

(72) Erfinder: **Kleemann, Axel, Dr. Dipl.-Chem., Greifenhagenstrasse 9, D-6450 Hanau 9 (DE)**
Erfinder: **Lüssling, Theodor, Dr. Dipl.-Chem., Zum Purren 5, D-7750 Konstanz (DE)**
Erfinder: **Pfeifer, Wolf, Dr. Dipl.-Chem., Stettiner Strasse 4, D-5040 Brühl (DE)**
Erfinder: **Scherberich, Paul, Dr. Dipl.-Chem., Königsberger Strasse 8, D-7750 Konstanz (DE)**

**EP 0 037 480 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von 5-Arylidenhydantoinen (A)

Gegenstand der Erfindung ist ein Verfahren zur Herstellung von im aromatischen Kern unsubstituierten oder substituierten 5-Arylidenhydantoinen durch Kondensation von entsprechenden unsubstituierten oder substituierten aromatischen Aldehyden mit Hydantoin in Gegenwart eines Amins.

5-Arylidenhydantoine sind wichtige Zwischenprodukte für die Herstellung von Phenylalanin und im aromatischen Kern substituierten Phenylalaninen.

Es ist bereits bekannt, 5-Arylidenhydantoine durch Kondensation von Benzaldehyd oder substituierten aromatischen Aldehyden mit Hydantoin in Gegenwart von Piperidin oder Diäthylamin und in Abwesenheit eines Lösungsmittels oder in Pyridin als Lösungsmittel herzustellen (Biochem. J. 29, Seiten 542 bis 545 [1935]). Abgesehen davon, daß die Ausbeuten von Aldehyd zu Aldehyd stark unterschiedlich sind, erfordern annehmbare Ausbeuten in jedem Falle eine unerwünscht lange Reaktionszeit.

Es ist auch bereits bekannt, die Kondensation des Aldehyds mit dem Hydantoin in Gegenwart von Essigsäure und von wasserfreiem Natriumacetat vorzunehmen (J. Amer. Chem. Soc. 45, Seiten 368 bis 383 [1911]). Bei der Umsetzung von Benzaldehyd betragen die Ausbeuten beispielsweise 70 bis 80%, bezogen auf eingesetztes Hydantoin. Zur Erzielung dieser Ausbeuten ist jedoch der Einsatz relativ großer Mengen an wasserfreiem Natriumacetat erforderlich. Wird die Menge des Natriumacetats vermindert, so geht die Ausbeute drastisch zurück.

Es ist ferner bereits bekannt, 5-(3',4'-Dioxybenzyliden)-hydantoin durch Kondensation von Protocatechualdehyd mit Hydantoin in Gegenwart von Natronlauge und wäßriger Ammoniumchlorid-Lösung herzustellen (DE-B-1 038 050, Beispiel 3). Das erhaltene Arylidenhydantoin ist jedoch gelb verfärbt und fällt in einer Ausbeute von nur 81,8% der Theorie an.

Es ist weiter bereits bekannt, 5-(3',4'-Dimethoxybenzyliden)-hydantoin durch Kondensation von 3,4-Dimethoxybenzaldehyd mit Hydantoin in wäßriger Lösung und in Gegenwart von Diäthanolamin oder Ammoniak herzustellen (Chem. Abstr. 82 [21], 1975, Referat 140 139v). Die Ausbeute, bezogen auf das eingesetzte Hydantoin, beträgt jedoch nur 80,6% der Theorie.

Es ist schließlich auch schon bekannt, im aromatischen Kern unsubstituierte oder substituierte 5-Arylidenhydantoine durch Kondensation der entsprechenden aromatischen Aldehyde mit Hydantoin in Gegenwart von Monoäthanolamin herzustellen (FR-A-2 189 040).

Das erfindungsgemäße Verfahren ist nun dadurch gekennzeichnet, daß man die Kondensation bei einer Temperatur zwischen 80 und 200°C in gleichzeitiger Gegenwart mindestens eines primären, sekundären oder tertiären Amins und mindestens einer aliphatischen oder aromatischen Carbonsäure vornimmt.

Das erfindungsgemäße Verfahren ergibt überraschenderweise auch schon bei Einsatz relativ geringer Mengen an Amin innerhalb tragbarer Reaktionszeiten gute Ausbeuten an dem gewünschten 5-Arylidenhydantoin. Außerdem fallen die 5-Arylidenhydantoine in hoher Reinheit an, was sich im Hinblick auf ihre Weiterverarbeitung zu den entsprechenden Phenylalaninen günstig auswirkt.

Das erfindungsgemäße Verfahren eignet sich besonders zur Herstellung von 5-Arylidenhydantoinen der allgemeinen Formel

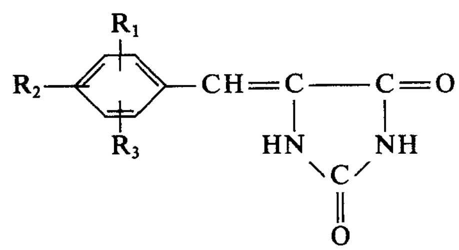

(I)

in der $R_1$, $R_2$ und $R_3$ gleich oder verschieden sind und jeweils Wasserstoff, Halogen, eine Hydroxylgruppe, eine Nitrogruppe, eine Aminogruppe, eine unverzweigte oder verzweigte Alkyl- oder Alkenylgruppe, eine Cycloalkylgruppe, eine Cycloalkenylgruppe, eine Arylgruppe, eine Aralkylgruppe, eine Alkarylgruppe, eine Alkoxygruppe, eine Alkylthiogruppe, eine Acyloxygruppe, eine Acylthiogruppe, eine Mono- oder Dialkylaminogruppe oder eine Acylaminogruppe bedeuten. Vorzugsweise enthalten die Alkylgruppen 1 bis 6, insbesondere 1 bis 3, Kohlenstoffatome, die Alkenylgruppen 2 bis 6, insbesondere 2 bis 3 Kohlenstoffatome, die Cycloalkyl- und Cycloalkenylgruppen 3 bis 8, vorzugsweise 3 bis 6 Kohlenstoffatome. Gegebenenfalls kann in den Cycloalkyl- und Cycloalkenylgruppen auch einmal oder mehrmals $-CH_2-$ durch $-O-$, $-S-$ oder $-NH-$ bzw. $-CH=$ durch $-N=$ ersetzt sein, so daß entsprechende heterocyclische Ringe mit 3 bis 8, insbesondere 3 bis 6 Ringgliedern vorliegen. Vorzugsweise enthalten die Aralkyl- und die Alkarylgruppen 1 bis 6, insbesondere 1 bis 3 Kohlenstoffatome in der Alkylen- bzw. Alkylgruppe. Die Alkoxy-, Alkylthio-, Acyloxy-, Acylthio-, Mono- oder Dialkylamino- und Acylaminogruppen enthalten vorzugsweise 1 bis 6, insbesondere 1 bis 3

Kohlenstoffatome in den Alkyl- bzw. Acylgruppen. Gegebenenfalls können auch zwei der Reste $R_1$ bis $R_3$ zusammen eine Alkylen- oder Alkenylengruppe mit 1 bis 6, insbesondere 1 bis 4 Kohlenstoffatomen bilden, wobei auch in diesem Fall einmal oder mehrmals $-CH_2-$ durch $-O-$, $-S-$ oder $-NH-$ bzw. $-CH=$ durch $-N=$ ersetzt sein kann.

Eingesetzt werden können demnach aromatische Aldehyde der allgemeinen Formel

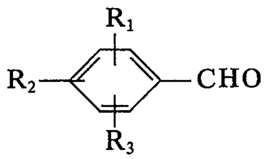

(II)

in der $R_1$, $R_2$ und $R_3$ die vorstehend angegebene Bedeutung haben. Solche Aldehyde sind beispielsweise Benzaldehyd, Tolylaldehyd, 4-Isopropylbenzaldehyd, 4-Hydroxybenzaldehyd, 3,4,5-Trimethoxybenzaldehyd, 3-Brom-4-methoxybenzaldehyd, 3,4-Methylendioxybenzaldehyd, 2-Hydroxy-4-nitrobenzaldehyd, 4,5-Dimethoxy-2-nitrobenzaldehyd, Salicylaldehyd, Vanillin, 4-Phenylbenzaldehyd, 4-Benzylbenzaldehyd, 4-Fluorbenzaldehyd, 4-Dimethylaminobenzaldehyd, 4-Acetoxybenzaldehyd, 4-Acetaminobenzaldehyd, 4-Methylthiobenzaldehyd und 3,5-Dichlor-4-hydroxybenzaldehyd.

Die Kondensation mit dem Hydantoin erfolgt in Gegenwart mindestens eines Amins. Als Amine kommen in Frage primäre Amine, wie Äthylamin, n-Propylamin, Monoäthanolamin, Anilin oder Benzylamin; sekundäre Amine, wie Dimethylamin, Diäthylamin, Piperidin, Diäthanolamin, Dibenzylamin, Morpholin oder Thiomorpholin; oder tertiäre Amine, wie Trimethylamin, Triäthylamin, N-Methylpiperidin, N-Methylpyrrolidin, Tri-n-propylamin, Triäthanolamin, N-Methylmorpholin, Benzyl-N,N-dimethylamin, Pyridin oder 4-Dimethylaminopyridin.

Die Kondensation des Aldehyds mit dem Hydantoin erfolgt ferner in Gegenwart mindestens einer aliphatischen oder aromatischen Carbonsäure. Geeignete Carbonsäuren sind beispielsweise Ameisensäure, Essigsäure, Propionsäure, Buttersäure, 2-Methylbuttersäure, Valeriansäure, Isovaleriansäure, 2-Chloressigsäure, 2-Hydroxyessigsäure, Benzoesäure, Mandelsäure, Phenylessigsäure, Zimtsäure, Phenylpropionsäure oder Crotonsäure.

Es kann vorteilhaft sein, die Kondensation in einem Lösungsmittel durchzuführen. Als solche kommen aliphatische oder aromatische Alkohole, aliphatische oder aromatische Äther, aliphatische oder aromatische Kohlenwasserstoffe, halogenierte aliphatische oder aromatische Kohlenwasserstoffe und deren Gemische in Frage. Beispielsweise werden n-Butanol, tert.-Butanol, Toluol, Benzylalkohol, Xylol oder Chlorbenzol eingesetzt.

Im allgemeinen erfolgt die Kondensation bei Temperaturen zwischen 80 und 200°C, insbesondere bei Temperaturen zwischen 100°C und der Siedetemperatur des Gemisches. Der Druck kann weitgehend beliebig gewählt werden. Es kann also bei Normaldruck wie auch bei niedrigerem oder höherem Druck gearbeitet werden. Obwohl es im allgemeinen vorteilhaft ist, bei etwa Normaldruck zu arbeiten, kann wegen der Flüchtigkeit der Substanzen erhöhter Druck erforderlich sein.

Das Mengenverhältnis von Aldehyd zu Hydantoin kann sowohl stöchiometrisch als auch unter- oder überstöchiometrisch gewählt werden. Im allgemeinen ist es vorteilhaft, je Mol Hydantoin etwa 1 bis 2 Mol, insbesondere 1,2 bis 1,5 Mol des Aldehyds einzusetzen.

Je Mol Hydantoin werden zweckmäßigerweise wenigstens 0,3 Mol, vorzugsweise 0,5 bis 2,0 Mol, insbesondere 0,8 bis 1,2 Mol des Amins eingesetzt.

Je Mol Hydantoin werden ferner zweckmäßigerweise wenigstens 0,3 Mol, vorzugsweise 0,5 bis 2,0 Mol, insbesondere 0,8 bis 1,2 Mol der Carbonsäure eingesetzt. Es kann vorteilhaft sein, mehr als 2,0 Mol Carbonsäure je Mol Hydantoin einzusetzen, wobei die überschüssige Carbonsäure gleichzeitig als Lösungsmittel dient.

Es kann weiterhin vorteilhaft sein, das bei der Umsetzung entstehende Wasser während der Reaktion durch Destillation abzutrennen.

## Beispiel 1

Ein Gemisch aus 100 g (1,0 Mol) Hydantoin, 127,5 g (1,2 Mol) Benzaldehyd, 85 g (1,0 Mol) Piperidin und 210 g (3,5 Mol) Essigsäure wurde 4 Stunden lang auf Rückflußtemperatur gehalten. Beim Abkühlen auf Raumtemperatur schied sich 5-Benzylidenhydantoin ab. Die Ausbeute betrug 169 g, entsprechend 90% der Theorie, bezogen auf eingesetztes Hydantoin. Das Produkt hatte einen Schmelzpunkt von 219–220°C. Es war, wie durch Dünnschichtchromatographie festgestellt wurde, einheitlich.

## Beispiel 2

Ein Gemisch aus 100 g (1,0 Mol) Hydantoin, 117 g (1,1 Mol) Benzaldehyd, 85 g (1,0 Mol) Piperidin, 60 g (1,0 Mol) Essigsäure und 200 ml n-Butanol wurde 5 Stunden lang auf Rückflußtemperatur gehalten.

Beim Abkühlen auf Raumtemperatur schied sich 5-Benzylidenhydantoin ab. Die Ausbeute betrug 175 g, entsprechend 93% der Theorie, bezogen auf eingesetztes Hydantoin. Das Produkt hatte einen Schmelzpunkt von 218–220°C. Es war, wie durch Dünnschichtchromatographie festgestellt wurde, einheitlich.

Beispiel 3

Es wurde wie im Beispiel 1 verfahren, jedoch wurden 101 g (1,0 Mol) Triäthylamin statt Piperidin eingesetzt. Die Ausbeute an 5-Benzylidenhydantoin betrug 167 g, entsprechend 89% der Theorie.

Beispiel 4

Es wurde wie nach Beispiel 2 verfahren, jedoch wurden 73 g (1,0 Mol) Diäthylamin statt Piperidin eingesetzt. Die Ausbeute an 5-Benzylidenhydantoin betrug 165,5 g, entsprechend 88% der Theorie.

Beispiel 5

Ein Gemisch aus 100 g (1,0 Mol) Hydantoin, 225 g (1,5 Mol) 3,4-Methylendioxybenzaldehyd, 135 g (1,0 Mol) N,N-Dimethylbenzylamin, 89 g (1,2 Mol) Propionsäure und 300 ml Xylol wurde 5 Stunden lang auf Rückflußtemperatur gehalten. Beim Abkühlen auf Raumtemperatur schied sich 5-(3',4'-Methylendioxybenzyliden)-hydantoin ab. Die Ausbeute betrug 202 g, entsprechend 87% der Theorie. Das Produkt hatte einen Schmelzpunkt von 244–245°C. Es war, wie durch Dünnschichtchromatographie festgestellt wurde, einheitlich.

Beispiel 6

Ein Gemisch aus 100 g (1,0 Mol) Hydantoin, 196 g (1,3 Mol) 4-Nitrobenzaldehyd, 186 g (1,3 Mol) Tri-n-propylamin und 240 g (4,0 Mol) Essigsäure wurden, wie in Beispiel 1 beschrieben, umgesetzt. Die Ausbeute an 5-(4'-Nitrobenzyliden)-hydantoin betrug 214 g, entsprechend 92% der Theorie. Das Produkt hatte einen Schmelzpunkt von 253–254°C. Es war dünnschichtchromatographisch einheitlich.

Beispiel 7

Ein Gemisch aus 100 g (1,0 Mol) Hydantoin, 119 g (1,1 Mol) 4-Methoxybenzaldehyd, 87 g (1,1 Mol) Pyridin, 72 g (1,2 Mol) Essigsäure und 250 ml Toluol wurde 6 Stunden lang auf Rückflußtemperatur erhitzt. Die Ausbeute an 5-(4'-Methoxybenzyliden)-hydantoin betrug 187,5 g, entsprechend 86% der Theorie. Das Produkt hatte einen Schmelzpunkt von 242–244°C.

Beispiel 8

Ein Gemisch aus 100 g (1,0 Mol) Hydantoin, 169 g (1,2 Mol) 2-Chlorbenzaldehyd, 74 g (0,75 Mol) 1-Methylpiperidin, 69 g (1,5 Mol) Ameisensäure und 150 ml Xylol wurde 3 Stunden lang auf Rückflußtemperatur erhitzt. Die Ausbeute an 5-(2'-Chlorbenzyliden)-hydantoin betrug 189 g, entsprechend 85% der Theorie.

Beispiel 9

Ein Gemisch aus 100 g (1,0 Mol) Hydantoin, 106 g (1,0 Mol) Benzaldehyd, 45 g (1,0 Mol) Äthylamin und 240 g (4,0 Mol) Essigsäure wurde wie in Beispiel 1 beschrieben umgesetzt. Es wurden 181 g 5-Benzylidenhydantoin, entsprechend 96% der Theorie, bezogen auf Hydantoin, erhalten.

Beispiel 10

Ein Gemisch aus 100 g (1,0 Mol) Hydantoin, 117 g (1,1 Mol) Benzaldehyd, 107 g (1,0 Mol) Benzylamin und 300 g (5,0 Mol) Essigsäure wurde wie in Beispiel 1 beschrieben umgesetzt. Die Ausbeute an 5-Benzylidenhydantoin betrug 179 g, entsprechend 95% der Theorie, bezogen auf Hydantoin.

## Patentansprüche

1. Verfahren zur Herstellung von im aromatischen Kern unsubstituierten oder substituierten 5-Arylidenhydantoinen durch Kondensation von entsprechenden unsubstituierten oder substituierten aromatischen Aldehyden mit Hydantoin in Gegenwart eines Amins, dadurch gekennzeichnet, daß man die Kondensation bei einer Temperatur zwischen 80 und 200° C in gleichzeitiger Gegenwart mindestens eines primären, sekundären oder tertiären Amins und mindestens einer aliphatischen oder aromatischen Carbonsäure vornimmt.

2. Verfahren nach Anspruch 1, dadurch gekennzeichnet, daß man das Amin in einer Menge von wenigstens 0,3 Mol pro Mol umzusetzenden Hydantoins einsetzt.

3. Verfahren nach Anspruch 1 oder 2, dadurch gekennzeichnet, daß man die Carbonsäure in einer Menge von wenigstens 0,3 Mol pro Mol umzusetzenden Hydantoins einsetzt.

## Claims

1. A process for the production of 5-arylidene hydantoins which are substituted or unsubstituted in the aromatic nucleus, by the condensation of corresponding substituted or unsubstituted aromatic aldehydes with hydantoin in the presence of an amine, characterised in that the condensation is carried out at a temperature of from 80 to 200° C in the simultaneous presence of at least one primary, secondary or tertiary amine and at least one aliphatic or aromatic carboxylic acid.

2. A process according to claim 1, characterised in that the amine is used in a quantity of at least 0.3 mols per mol of hydantoin to be reacted.

3. A process according to claim 1 or 2, characterised in that the carboxylic acid is used in a quantity of at least 0.3 mols per mol of hydantoin to be reacted.

## Revendications

1. Procédé pour la fabrication de 5-arylidène-hydantoînes non-substituées, ou substituées sur le noyau aromatique, par condensation des aldéhydes aromatiques correspondants, non substitués ou substitués, avec l'hydantoîne en présence d'une amine, procédé caractérisé en ce qu'on effectue la condensation avec présence simultanée d'au moins une amine primaire, secondaire ou tertiaire et d'au moins un acide carboxylique aliphatique ou aromatique, à une température qui se situe entre 80 et 200° C.

2. Procédé suivant la revendication 1, caractérisé en ce que l'on met en oeuvre l'amine dans une proportion d'au moins 0,3 mole par mole d'hydantoîne à condenser.

3. Procédé suivant l'une des revendications 1 et 2, caractérisé en ce que l'on met en oeuvre l'acide carboxylique dans une proportion d'au moins 0,3 mole par mole d'hydantoîne à condenser.